Europäisches Patentamt

European Patent Office

Office européen des brevets

(11) Publication number: **0 198 662**
**A1**

(12) **EUROPEAN PATENT APPLICATION**

(21) Application number: **86302632.4**

(22) Date of filing: **09.04.86**

(51) Int. Cl.⁴: **C 12 Q 1/68**

(30) Priority: **12.04.85 GB 8509367**

(43) Date of publication of application: **22.10.86**
**Bulletin 86/43**

(84) Designated Contracting States: **DE FR GB**

(71) Applicant: **AMERSHAM INTERNATIONAL plc, Amersham Place, Little Chalfont Buckinghamshire HP7 9NA (GB)**

(72) Inventor: **Miles, Vincent Joseph, 1005 South Prairie Avenue, Barrington Illinois 60010 (US)**

(74) Representative: **Pennant, Pyers et al, Stevens, Hewlett & Perkins 5 Quality Court Chancery Lane, London, WC2A 1HZ (GB)**

(54) **Method for nucleic acid hybridisation.**

(57) A method of detecting a specific target polynucleotide sequence in a sample uses: a labelled first probe and a second probe, both probes to hybridise to different sequences of the target; and a solid phase carrying groups to bind the second probe. The method comprises contacting the sample under hybridising conditions with solutions of both probes, preferably in excess. Thereafter the mixture is contacted with the solid phase which is then washed. Target attached to the solid phase can be detected by means of the labelled first probe. The solid phase may be a dipstick carrying streptavidin to bind with the biotin groups attached to the second phase.

- 1 -

## Method for Nucleic Acid Hybridisation

### Introduction

There is increasing interest in the use of nucleic acid hybridisation for diagnostic purposes in clinical laboratories. A nucleic acid probe specific for a particular pathogen or group of pathogens can be used to detect the presence of the pathogen(s) in clinical samples which have been processed appropriately.

Current methods for nucleic acid hybridisation were largely developed in research laboratories and are not ideal for clinical applications. In this invention, a method of hybridisation is proposed which would be much more suitable for such applications; it could easily, for instance, be adapted to provide a "dipstick" procedure for detecting the formation of hybrids.

Before describing the proposed method, currently available methods will be reviewed briefly, with an assessment of their advantages and disadvantages.

### Currently Available Assays

For the purpose of the ensuing discussion, hybridisation methods will be divided into two classes: those in which the "target" nucleic acid is immobilised on a solid support, and those in which it is in solution. For the sake of textual clarity, the discussion will assume the use of a DNA probe to detect a DNA target; either or both of the probe and target could of course be RNA rather than DNA.

(a) Target immobilised.

The great majority of research hybridisation assays depend on this type of methodology. In these assays, the target DNA is denatured into single strands which are then bound to a solid support such as a membrane filter made of nitrocellulose or nylon. To perform the hybridisation, this filter is immersed in a solution containing labelled probe. After incubation for an appropriate period, the filter is taken out of the probe solution and washed thoroughly to remove any probe which did not bind specifically to the target DNA. The presence of labelled probe bound to the filter can then be detected by methods appropriate for the label used.

This type of assay has a number of advantages:
- Because the separated strands of the target DNA are immobilised, they cannot re-anneal with each other in a reaction that would compete with the binding of labelled probe.
- As in all solid-phase assays, separation of bound from unbound probe can be achieved very easily.
- Many samples can be "spotted" onto a single sheet of membrane filter and processed simultaneously.
- If required, topographical information present in the original sample can be preserved. Thus, the relative positions of electrophoretically-separated fragments of DNA or of intracellular components can be maintained in, respectively, "Southern blot" or in situ hybridisations.

Two disadvantages of this type of assay are:
- Time is required to process the sample in such a way that the target DNA will bind to the solid phase.
- More seriously, the kinetics of the hybridisation reaction (binding of probe to target DNA) are adversely affected by having one of the partners

- 3 -

in this reaction bound to a solid phase rather than in solution. To a certain extent, this problem can be overcome by using a large excess of probe.

Six common types of assay in this category are:
Southern blot: Southern E.M., (1975) J. Mol., Biol., $\underline{98}$ 503-517.
Northern blot: Alwine J.C., Kemp D.J., and Stark G.R., (1977) Proc., Natl., Acad., Sci., U.S.A., $\underline{74}$, 5350-5354.
Dot blot (spot blot): Brandsma I., and Miller G., (1980) Proc., Natl., Acad., Sci., U.S.A., $\underline{77}$ 6851-6855.
In situ hybridisation: Gall J.G., and Pardue M.L., (1969) Proc., Natl., Acad., Sci., U.S.A, $\underline{63}$ 378-383.
Colony hybridisation: Grunstein M., and Hogness D.S., (1975) Proc., Natl., Acad., Sci., U.S.A., $\underline{72}$ 3961-3965.
Plaque hybridisation: Benton W.D., and Davis R.W., (1977) Science $\underline{196}$, 180-182.

(b) Target in solution.

In its simplest form, this type of assay involves mixing denatured target DNA with probe DNA in solution, and monitoring the formation of hybrids between target and probe. In practice, however, it can be difficult to separate bound from unbound probe. One approach, for instance, is to separate hybrids from unhybridised material by binding them to hydroxyapatite (which has a higher affinity for double-stranded than for single-stranded nucleic acids). Aside from the fact that the separation achieved may not always be absolute, this approach suffers from the inherent disadvantage that it relies on a physical property -- double-strandedness -- which is not exclusive to the hybrid; the probe may become involved in other double-stranded structures which do not involve the target sequence, such as those formed by self-annealing. This places unnecessary restrictions on the type of probe which can be used.

More promising for clinical purposes is the "sandwich hybridisation" assay [Dunn A. R. and Hassell J. A. (1977) Cell, 12, 23-36; Virtanen M., Laaksonen M., Soderlund H., Palva A., Halonen P. and Ranki M. (1983) The Lancet, Feb., 19, 1983, 381-383; and EPA 139489] which as its name suggests is the DNA version of one of the popular types of immunoassay. In sandwich immunoassays, two antibodies are required, each recognising a different epitope (determinant) of the target antigen; similarly, in sandwich hybridisations, two different DNA probes are required, each hybridising to a different sequence in the target DNA. In its present state of development, the sandwich hybridisation assay has one of the probes bound, unlabelled, to a solid phase where it acts as a "capture agent" for any target DNA present in solution; the second, labelled, probe is also present in solution and only becomes bound to the solid phase if target DNA has been captured by the first probe.

To date, very little has been published on the use of sandwich hybridisation, which means that it is too early for the advantages and disadvantages of the technique to have emerged clearly. From a theoretical standpoint, however, one can speculate that they will be as follows:-

Compared with the immobilised-target type of assay, the sandwich hybridisation procedure should have one main advantage:-

- Sample preparation should be less time-consuming because there is no need to immobilise the target DNA on a solid phase.

In its present form, however, this type of assay could have several disadvantages:-

- A major problem could be that the assay is sample-driven rather than reagent-driven; this could make it very difficult to "force along" the hybridisation reaction to give a reproducibly rapid and sensitive result. The reason for this is that the assay still features a kinetically undesirable reaction between a solid-phase partner (the first probe) and a solution-phase partner (the target). In the immobilised-target type of assay, this problem can be addressed by using an excess of solution-phase partner, i.e. probe DNA. The same approach is not really possible in a sandwich hybridisation, because the solution-phase partner is the target DNA, whose concentration will depend on the nature of the sample.

- The separated strands of the target DNA are free to re-anneal with each other rather than to the probe DNAs; in theory at least, this will reduce the sensitivity of this type of assay. The re-annealing process could be slowed by reducing the concentration of the target DNA; as has been shown, however, this would be very undesirable in a sample-driven hybridisation assay.

The Invention

The invention provides a method of performing nucleic acid hybridisations which should combine the advantages of the two approaches so far discussed while eliminating their disadvantages.

The reasoning behind this method is as follows. Hybridisations are most effective and sensitive when the probe and target sequences are both in solution, and (each) probe is present in excess over the target so as to drive the reaction. Separation and detection of hybrids, on the other hand, can be achieved most successfully when the hybrids are immobilised onto a solid phase. What is needed, therefore, is a method which allows a "phase-change" between hybridisation and separation/detection.

In the method, this phase-change is achieved by performing a sandwich hybridisation in which both of the probes are in solution; one of them is, however, modified in such a way that it can easily be bound to a solid phase once the hybridisation is complete.

Thus the invention provides a method of detecting a specific target polynucleotide sequence in a sample, by the use of

a) a first polynucleotide probe having a single-stranded sequence complementary to a sequence of the target, and which is labelled,

b) a second polynucleotide probe having a single-stranded sequence complementary to a different sequence of the target, and

c) a solid phase, wherein the solid phase c) and the second probe b) carry groups reactive with one another to cause b) and c) to become bound together when the two are brought into contact,

which method comprises the steps of

i) contacting the sample in solution under hybridising conditions with solutions of the probes a) and b),

ii) contacting the resulting mixture with the solid phase c) under conditions to cause the probe b) to become bound thereto, and

iii) observing the presence or absence of the label of the first probe a) bound to the solid phase.

The first probe a) is labelled. By this is meant that the probe is isotopically or chemically modified in such a way that the person performing the hybridisation can, after further manipulation if necessary, detect the presence of the first probe. The nature of the label is not critical; it may be a radioactive atom or a component of an enzyme or fluorescent or chemiluminescent system, or merely a chemical group by means of which such a component may subsequently be added. Techniques for labelling polynucleotide probes are well known and will not be described here. The single-stranded sequence of the first probe that is complementary to the target sequence may, but need not be, labelled. The labelled part of the first probe can be partly or wholly double-stranded. A requirement of the label is that it should not lead to non-specific binding of the first probe a) to the solid phase c), the second probe b) or the sample.

A variety of methods can be used to modify the second probe, provided that the method used allows the second probe b) to be attached to the solid phase c) after the hybridisation, but it does not generate any signal in the procedure used to detect hybridised first probe a), and that it does not lead to non-specific binding between the second probe b) and either the sample or the first probe a). For example, the second probe b) may carry biotin groups and the solid phase c) may carry streptavidin. Or the second probe may carry biotin or some other hapten and the solid phase may carry a corresponding antibody. Techniques for attaching complementary reactive groups to polynucleotide probes and to solid phases are well known in the art and will not be described here.

The shape of the solid phase is not critical, and a variety of massive or particulate configurations may be used. One example is latex particles which may be dispersed in the hybridisation mixture and which may contain a magnetizable material for easy subsequent separation. Another example is a microtiter plate. A third example is a dipstick which may be dipped in the hybridisation mixture and subsequently removed and washed prior to observing the label.

It is necessary, and is inherent in the method, that the two probes should hybridise to different regions of the target polynucleotide sequence. The sample may be hybridised with the two probes, either together, or separately in either order. It is an advantage of the invention that, being in solution, the two probes can be used in excess, so as to drive the hybridisation reaction in the forward direction. After incubation for a sufficient time to permit hybridisation of the two probes with the target polynucleotide sequence (if present), the solid phase is added and the reaction conditions adjusted if necessary to effect binding between the probe b) (and any hybrids in which it is involved) and the solid phase. Then the solid phase may be separated by known means from the hybridisation solution, washed, and further treated by known means to observe the presence of label thereon as indicating the presence in the sample of the target polynucleotide sequence.

Thus, in the case where the first probe carries a radioactive label, the second probe carries biotin groups, and the solid phase is a dipstick coated with a material to which is bound streptavidin, the method may comprise the following steps:-

- Mix the sample in solution with an excess of each of the two probes,
- incubate this mixture to allow hybridisation to occur,
- bring the mixture into contact with the dipstick carrying immobilised streptavidin, and allow the biotinylated second probe (and any hybrids in which it is involved) to bind to the dipstick,
- recover and wash the dipstick, and then measure the amount of radioactivity bound to it - there will be little if any bound if the sample did not contain the target polynucleotide sequence.

The advantages of the method have already been alluded to: the most important of them are:-

a) speed and sensitivity resulting from reaction in solution involving the use of an excess of each of the two probes to drive the hybridisation reaction; this excess should also minimize any problems due to re-anealing of target polynucleotide sequences.

b)   easy separation and detection of hybrids.

c)   easy adaptation to formats suitable for use in clinical laboratories, for example involving a dipstick.

### Experimental

The practice of the invention can be illustrated using a model system based on plasmid pAT153, cosmid pJB8 and bacteriophage λ Charon 4A.   pJB8 DNA contains plasmid DNA sequences derived from pAT153 and λ DNA sequences derived from Charon 4 (Ish-Horowicz, D. and Burke, J.G., Nucleic Acids Research, volume 9, page 2989ff, 1981); it should therefore hybridise with both a pAT153 DNA probe and a Charon 4 DNA probe, whereas these two probes should not hybridise directly with one another.   (For convenience, it is easier to use a Charon 4A DNA probe than a Charon 4 DNA probe, because Charon 4A DNA is commercially available; the two phages are identical in the region which is represented in pJB8).

In the model system, denatured pJB8 "target" DNA is hybridised in solution with a biotin-labelled pAT153 DNA probe and a 32P-labelled Charon 4A DNA probe, hybrids are captured using beads to which streptavidin had been linked covalently, the beads are washed to remove the labelled material not involved in specific hybrids, and the amount of bound 32P is determined by liquid scintillation counting.

### Restriction digests of pAT153 and pJB8 DNAs

Typical preparations of plasmid and cosmid DNA contain  significant proportions of highly supercoiled covalently closed circular (ccc) DNA.   To ensure that the pAT153 and pJB8 target DNAs used in this experiment could be completely denatured prior to their immobilisation on a nylon hybridisation member, each DNA was converted from ccc to linear form by digestion with restriction endonuclease Bam HI. In each case, 1 $\mu$g of DNA was digested to completion under standard conditions for this enzyme (37°C, in 50mM Tris-HCl pH 7.4, 10mM $MgSO_4$, 100mM NaCl) and the reaction was terminated by heat inactivation of the enzyme at 65°C.

### Preparation of labelled probes

Samples of Charon 4A DNAs were labelled with phosphorus-32 by nick translation using a commercially available kit and [$\alpha$-32P]dCTP (from Amersham International). 1$\mu$g of the DNA was labelled to a specific activity of approximately 4 X $10^7$ dpm/$\mu$g using "procedure A" from the protocol booklet supplied with the kit.

The nick translation reaction was terminated by the addition of EDTA to a concentration of 10mM. The labelled probe was then separated from unincorporated nucleotides by gel filtration on a 10 X 1 cm column of Sephadex G50, the probe was eluted in TE buffer (10mM Tris-HCl, pH 8.0, 1mM EDTA) in a volume of approximately 2 ml and stored frozen at -20°C.

### Labelling of DNA by nick translation

Nick translations were performed using the following reagents:-
- Nick translation kit}           All from
- [1', 2', 5-3H]dCTP}     Amersham International
- biotin-11-dUTP: synthesised according to the method of Ward and his colleagues (Brigati, D.J., et al, Virology, volume 126, pages 32-50, 1983) and used as 0.3mM solution.

These reagents were used to set up a nick translation reaction containing 1ug pAT153 DNA and each of the nucleotides dATP, dCTP, dGTP and biotin-11-dUTP at a concentration of 20µM. The unlabelled dCTP was supplemented with 5µCi of [3H]dCTP, simply to permit the incorporation of nucleotides into DNA to be monitored.

The nick translation was allowed to proceed for 3 hours at 15°C, after which the reaction was terminated and the labelled DNA separated from unincorporated nucleotides as described above. From the quantity of tritium incorporated into DNA, it could be calculated that about 14% of the T residues had been replaced with bio-dU; the DNA from this reaction will henceforth be referred to as bio/3H-pAT.

Preparation of streptavidin-coated beads

Two samples of streptavidin-coated beads were used.

The first, henceforth referred to as PA/SA beads, consisted of polyacrolein beads to which streptavidin had been linked as follows. The beads used had a mean diameter of 1.37 µm. 20 mg beads were washed three times in 0.1M carbonate/bicarbonate buffer, pH 9.5, and resuspended in 1.0 ml of this buffer. 1.3 mg of streptavidin at a concentration of 5mg/ml in 10mM phosphate buffer pH 6 was added, and the mixture incubated for 2 hours at ambient temperature on a spiralling mixer. Next, 100 µl of a 5mg/ml solution of sodium borohydride (Sigma) was added to block unreacted aldehyde groups, and incubation continued at ambient temperature for a further two hours. The beads were then washed four times with 1ml of PBS containing 0.1% (w/v) Tween 20 [PBS (phosphate-buffered saline): 0.1M NaCl, 80 mM disodium hydrogen phosphate, 19mM sodium dihydrogen phosphate pH 7.4], once with 1ml of 1M NaCl, and once with PBS/0.1% Tween 20. Finally, the beads were stored at 4°C in 1 ml of PBS/0.1% Tween 20 containing 1% (w/v) BSA and 0.5% sodium azide.

The second sample of streptavidin-coated beads, henceforth referred to as a Amerlex-M/SA beads, consisted of magnetic Amerlex-M beads to which streptavidin had been attached as follows. Carboxylated Amerlex-M beads (carboxylated polystyrene latex beads with a magnetic core), mean diameter 1-2 µm, were obtained from Amersham International plc. 15mg of beads were washed three times in 10 mM phosphate buffer pH 6, and resuspended in 1 ml of the same buffer. To the beads were added 0.5 mg of streptavidin at a concentration of 6 mg/ml in 50 mM phosphate buffer pH 7.4, followed by 5 mg of 1-ethyl-3-(3-dimethylaminopropyl) carbodiimide hydrochloride at a concentration of 10 mg/ml in 10 mM phosphate buffer pH 6. The mixture was incubated at ambient temperature on a spiralling mixer for 2 hours, and the beads were then washed using the same sequence of washed described above for the PA/SA beads, except that the fifth wash was with 0.1M glycine pH 3.5 rather than with 0.1M NaCl, and was prolonged for 20 minutes. Finally, the beads were stored in 1 ml of PBS/0.1% Tween 20 containing 1% BSA and 0.5% sodium azide.

Example 1

This example comprised three experiments.

The first experiment demonstrated that pAT153 DNA and Charon 4A DNA both hybridise to pJB8 target DNA but not to each other.

The second experiment demonstrated that PA/SA beads can efficiently capture biotinylated DNA and hybrids containing biotinylated DNA, and that non-specific binding of non-biotinylated DNA is very low.

In this third experiment, the model system was implemented in its entirety: biotinylated pAT 153 DNA and 32P-labelled    Charon 4A DNA were hybridised in solution to samples containing various amounts of pJB8 DNA, any hybrids formed were captured on PA/SA beads, and the amount of 32P radioactivity which became attached to the beads was determined by LSC (liquid scintillation counting).

Ten solution hybridisation reactions were performed, consisting of five duplicate pairs. Reactions 1-4 were the model tests, and thus included the two probes (bio/3H-pAT and 32P-Ch4A) and various amounts of target pJB8 DNA.    Reactions 5-10 were controls, 5 and 6 featured a pAT153 target (to which 32P-Ch4A should not bind), 7 and 8 lacked bio/3H-pAT (required for binding of hybrids to streptavidin-coated beads), and 9 and 10 contained no target DNA at all.

Table 1 below summarises which DNAs were included in which reactions, and in what quantity.    One point worth commenting on is the large excess of 32P-Ch4A used in each reaction:    30 ng, compared with 2.5 ng of bio/3H-pAT.    The reason for this was that only about 1/30th of the Charon 4A genome is represented in pJB8, and so only about 1/30th of the input 32P-Ch4A was capable of hybridising to the pJB8 target.

The simplicity of the protocols used to perfrom the hybridisations and to assess the results provided a striking illustration of the utility of the invention. For each hybridisation, the DNAs to be included in the reaction were mixed together in a microcentrifuge tube in a total volume of 70 µl TE buffer, denatured by heated at 95°C for 10 minutes, and cooled immediately by placing the tubes on ice.    Concentrated stock solutions of SSC and SDS were added to give final concentrations of 6 X SSC (0.9M Nacl, 0.09M sodium citrate) and 0.1% SDS (sodium dodecyl sulphorate) and a final volume of 100 µl.    The tube was incubated overnight at 65°C, then placed on ice for 2 minutes.    To capture any hybrids formed, 25 μl of the stock suspension of PA/SA beads were added, and the mixture was placed at ambient temperature for 30 minutes, with occasional vortex mixing.    The beads were collected by centrifugation in a microfuge, and then washed, with washes 1 and 2 lasting for 20 minutes each, and washes 3 and 4 for 30 minutes each.    After the final wash the beads were again collected by centrifugation and the amount of radioactivity bound to them was determined by the standard LSC procedures which allowed separate quantitation of 3H (associated with bio-3H/pAT) and 32P (associated with 32P-Ch4A).

The experimental design and results obtained are summarised in Table 1.

## Table 1

### Test of model system using PA/SA beads

| Reaction number | Quantities of DNA included | | | | Radioactivity bound to PA/SA beads cpm | |
|---|---|---|---|---|---|---|
| | pJB8 target | pAT153 target | bio/3H-pAT probe | 32P-Ch4A probe | 3H | 32P |
| 1 | 500pg | | 2.5ng | 30ng | 2024 | 2939 |
| 2 | 500pg | | 2.5ng | 30ng | 2090 | 2587 |
| 3 | 50pg | | 2.5ng | 30ng | 1919 | 444 |
| 4 | 50pg | | 2.5ng | 30ng | 2014 | 525 |
| 5 | | 500pg | 2.5ng | 30ng | 2084 | 270 |
| 6 | | 500pg | 2.5ng | 30ng | 1585 | 80 |
| 7 | 500pg | | | 30ng | 0 | 19 |
| 8 | 500pg | | | 30ng | 0 | 46 |
| 9 | | | 2.5ng | 30ng | 1942 | 112 |
| 10 | | | 2.5ng | 30ng | 2036 | 136 |

These results demonstrate conclusively that the invention works in practice. The quantity of 32P-labelled Charon 4A which became attached to the PA/SA beads was significantly greater following hybridisations in which pJB8 target DNA was present (reactions 1-4) than the background level seen when any important component of the system was left out, as for instance when the target DNA was pAT153 rather than pJB8 (reactions 5 and 6), or when the biotinylated pAT153 probe was left out (reactions 7 and 8), or when no target DNA at all was present (reactions 9 and 10).

## Example 2

A preliminary experiment demonstrated that Amerlex-M/SA beads can efficiently capture biotinylated DNA and hybrids containing biotinylated DNA, and that non-specific binding of non-biotinylated DNA is very low.

## Model system featuring Amerlex-M/SA beads

This Example is essentially a repeat of Example 1, but with hybrid capture by Amerlex-M/SA beads rather than PA/SA, and with a new batch of $32P-Ch4A$ probe (labelled by nick translation to $4.8 \times 10^7$ dpm/$\mu$g), of which 75ng rather than 30ng was used per hybridisation. Reactions were set up as described in Example 1. After the overnight incubation, each reaction was placed on ice for 2 minutes, and 25 $\mu$l added of the stock suspension of Amerlex-M/SA beads. Following 30 minutes incubation at ambient temperature, beads were collected using a magnetic separator and then taken through the standard sequence of washes. Finally, the radioactivity bound to the beads was determined by standard LSC procedures; in this experiment, colour-quenching by the Amerlex-M beads significantly affected the measurement of bount 3H.

The experimental design and results are summarised in Table 2.

## Table 2

### Test of model system using Amerlex-M/SA beads

| Reaction number | Quantities of DNA included | | | | Radioactivity bound to Amerlex-M/SA beads (cpm) | |
| | pJB8 target | pAT153 target | bio/3H-pAT probe | 32P-Ch4A probe | 3H | 32P |
|---|---|---|---|---|---|---|
| 1 | 500pg | | 2.5ng | 75ng | 1200 | 1047 |
| 2 | 500pg | | 2.5ng | 75ng | 1480 | 1279 |
| 3 | 50pg | | 2.5ng | 75ng | 1394 | 221 |
| 4 | 50pg | | 2.5ng | 75ng | 1430 | 234 |
| 5 | | 500pg | 2.5ng | 75ng | 1310 | 67 |
| 6 | | 500pg | 2.5ng | 75ng | 1465 | 145 |
| 7 | 500pg | | | 75ng | 0 | 67 |
| 8 | 500pg | | | 75ng | 11 | 61 |
| 9 | | | 2.5ng | 75ng | 1443 | 155 |
| 10 | | | 2.5ng | 75ng | 1401 | 78 |

The conclusions from this experiment with Amerlex-M beads are exactly the same as those from the experiment with PA/SA beads described in the previous Example.

## C L A I M S

1.   A method of detecting a specific target polynucleotide sequence in a sample, by the use of

a)   a first polynucleotide probe having a single-
stranded sequence complementary to a sequence of the
target, and which is labelled,

b)   a second polynucleotide probe having a single-
stranded sequence complementary to a different sequence
of the target, and

c)   a solid phase, wherein the solid phase c) and
the second probe b) carry groups reactive with one
another to cause b) and c) to become bound together
when the two are brought into contact,

which method comprises the steps of

i)   contacting the sample in solution under
hybridising conditions with solutions of the probes a)
and b),

ii)   contacting the resulting mixture with the
solid phase c) under conditions to cause the probe b)
to become bound thereto, and

iii)   observing the presence or absence of the label
of the first probe a) bound to the solid phase.

2.   A method as claimed in claim 1,
wherein the second probe b) carries biotin groups and
the solid phase c) carries streptavidin.

3.   A method as claimed in claim 1,
wherein the second probe b) carries a hapten and the
solid phase c) carries a corresponding antibody.

4.   A method as claimed in any one of claims 1 to 3,
wherein the solid phase c) is in the form of a dipstick
which is dipped in the hybridisation mixture and
subsequently removed and washed prior to observing the
label.

5. A method as claimed in any one of claims 1 to 4, wherein each of the two probes is used in excess over the target.

6. A method as claimed in any one of claims 1 to 5, wherein the second probe b) carries biotin groups, the solid phase c) is in the form of a dipstick carrying streptavidin, and steps i) to iii) are:

i) contacting the sample in solution under hybridizing conditions with solutions of probes a) and b) in excess over the target,

ii) contacting the resulting mixture with the dipstick c) under conditions to cause the biotinylated probe b) to become bound thereto, and

iii) observing the presence or absence of the label of the first probe a) bound to the solid phase.

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,P | EP-A-0 159 719  (ENZO BIOCHEM INC.) <br> * Page  7,  lines 1-26; page 10, line  13 - page 12, line 23; page 16,  line  10  - page 18, line 4; claims 8-11 * | 1-3,5 | C 12 Q    1/68 |
| Y | | 4,6 | |
| X,P | EP-A-0 145 356  (NATIONAL RESEARCH DEVELOPMENT CORP.) <br> * Abstract; page 4, line 1 - page 5,  line 15; page 6, lines 14-24; page 8, lines 11-28 * | 1 | |
| Y | | 4,6 | |
| X,P <br> D | EP-A-0 139 489  (ORTHO DIAGNOSTIC SYSTEMS INC.) <br> * Figure  1;  abstract;  page 3, line  15  - page 6, line 29; page 7, lines 24-29 * | 1-3 | **TECHNICAL FIELDS SEARCHED (Int. Cl.4)** <br><br> C 12 Q <br> G 01 N |
| A | | 5 | |
| A | EP-A-0 130 515  (MOLECULAR DIAGNOSTICS) <br> * Abstract;  page  11, line 26 - page 13, line 14 * | 1 | |
| | --- | -/- | |

The present search report has been drawn up for all claims

| Place of search | Date of completion of the search | Examiner |
|---|---|---|
| THE HAGUE | 16-07-1986 | HITCHEN C.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82

European Patent Office

**EUROPEAN SEARCH REPORT**

| | **DOCUMENTS CONSIDERED TO BE RELEVANT** | | Page 2 |
|---|---|---|---|
| **Category** | Citation of document with indication, where appropriate, of relevant passages | Relevant to claim | **CLASSIFICATION OF THE APPLICATION (Int. Cl.4)** |
| A | EP-A-0 128 018 (ORGENICS LTD.) <br> * Page 5, line 4 - page 7, line 15; page 14, line 18 - page 15, line 11 * | 1-3,6 | |
| A | EP-A-0 128 332 (ENZO BIOCHEM INC.) <br> * Page 12, line 5 - page 16, line 10; page 19, line 13 - page 20, line 19; page 26, lines 18-24 * | 1-3,6 | |
| | | | **TECHNICAL FIELDS SEARCHED (Int. Cl.4)** |

The present search report has been drawn up for all claims

| Place of search | Date of completion of the search | Examiner |
|---|---|---|
| THE HAGUE | 16-07-1986 | HITCHEN C.E. |